# EUROPEAN PATENT APPLICATION

(11) **EP 4 304 163 A1**
(43) Date of publication of application: **10.01.2024**
(21) Application number: 22183580.4
(22) Date of filing: 07.07.2022
(51) Int. Cl.: H04N 5/32, A61B 6/00, G01T 1/24

(54) **VOLTAGE GENERATOR AND X-RAY IMAGING SYSTEM**

(71) Applicant: Koninklijke Philips N.V., 5656 AG Eindhoven (NL)
(72) Inventor: PROKSA, Roland, Eindhoven (NL)
(74) Representative: Philips Intellectual Property & Standards

(57) **Abstract**

The invention provides a voltage generator (1) for an X-ray imaging system (4), the voltage generator (1) comprising a trigger system (2) configured to, in response to receiving a start cycle trigger, trigger the voltage generator (1) to start transitioning to a first value of an output voltage of the voltage generator (1), and, in response to determining that the output voltage passes a predetermined threshold, trigger a start of a data acquisition of the detector (6) for a first integration period of a data acquisition cycle of the detector (6).

## Description

### FIELD OF THE INVENTION

The invention provides a voltage generator, an X-Ray imaging system, a method, a computer program product, and a computer readable medium.

### BACKGROUND OF THE INVENTION

X-ray imaging systems often employ kVp switching as image acquisition mode, where kVp refers to the output voltage of a voltage generator, which provides operating power for the radiation source of the imaging system.

In this imaging mode, the output voltage kVp is alternated rapidly between low and high kVp to acquire successive projections with different spectra, e.g., different X-ray spectra.

For example, during an acquisition cycle of detector of an imaging system, the voltage generator may switch or transition between two voltage values, i.e., a high voltage and a low voltage. A cycle may comprise, for example, at least a high voltage period and a low voltage period. A trigger signal, e.g., from an angular encoder, may be used to start the rising and falling transition of the output voltage.

Quality depends on having a well-defined and stable synchronization of the output spectrum of the radiation source and the data acquisition. Variations of the output spectrum, e.g., the X-ray flux, will cause a change of the effective spectrum of the acquired data. The spectral processing, e.g., for material decomposition, depends on the spectrum and is very sensitive to differences between the assumed and real spectra, and, accordingly, to variations of the spectrum

The temporal behavior of subsystems of the imaging system may, however, cause significant variations. This in turn can cause artifacts in the processing of the data. As an example, there are system components that typically have an uncertainty in the temporal behavior, also referred to as jitter.

As an example, a voltage generator may comprise a resonator and a transformer to generate voltage, e.g., high AC voltage. An energy injector may be provided, configured to inject energy into the resonator to increase the output voltage. When no energy is injected, the output voltage will drop consequently due to the output load of the X-Ray tube. A current switch may be used for the energy injection and may allow changes of switch states only during zero-crossing of the resonator. Accordingly, the generator may react only to an external trigger at discrete points in time, e.g., the zero crossings. As the phase and frequency of the resonator can hardly be controlled dynamically and synchronized with the cycle trigger, an inaccuracy, e.g., jitter, of the temporal reaction may occur. As an example, a generator having a mean resonance frequency of 100 kHz may show a random reaction time between J_{Gen} = 0 to 5 µs.

Thus, present systems are faced with the adverse impacts of uncertainty in temporal behavior.

### SUMMARY OF THE INVENTION

It is an object of the invention to allow for mitigating the adverse impacts of uncertainty in temporal behavior, e.g., jitter.

The invention provides a voltage generator, an X-ray imaging system, a method, a computer program product, and a computer readable medium according to the independent claims. Preferred embodiments are laid down in the dependent claims.

The invention provides a voltage generator for an X-ray imaging system, the voltage generator comprising a trigger system. The trigger system is configured to, in response to receiving a start cycle trigger, trigger the voltage generator to start transitioning to a first value of an output voltage of the voltage generator. The trigger system is further configured to trigger, in response to determining that the output voltage passes a predetermined threshold, a start of a data acquisition of a detector for a first integration period of a data acquisition cycle of the detector.

Thus, the start cycle trigger may cause the start of a data acquisition cycle of a detector. At the same time, the data acquisition may be closely linked to the actual output voltage of the voltage generator, in particular, the integration period may be more precisely synchronized with the voltage transition.

Accordingly, the trigger system as described above allows for a well-defined synchronization of the data acquisition and the voltage generator and, accordingly, the output spectrum of a radiation source powered by the voltage generator.

It is noted that the data acquisition by a detector, for example in imaging systems, e.g., a CT system, may be structured/divided into successive integration periods, IPs, of the detector. For example, the impinging X-ray flux to each detector pixel may be converted and integrated during one IP to form one acquisition data sample, e.g., associated to one projection.

The transitions between high and low value of the output voltage are particularly problematic in terms of contaminating high and low spectra with intermediate spectra. As such, using the present trigger system, which triggers acquisition based on output voltage values passing a predetermined threshold, allows for mitigating the contaminating effect of intermediate spectra.

Accordingly, the above-described challenges are overcome. In particular, triggering the data acquisition dependent on the actual output voltage allows for accounting for any delays during ramping up or down the output voltage. For example, the rise and fall of the voltage signal may conventionally introduce temporal uncertainties, which can be eliminated using the trigger system of the present disclosure.

Thus, the present disclosure may provide a triggering scheme to reduce the impact of jitter during kVp switching, e.g., spectral CT kVp switching, particularly when detector integrations also capture X-ray flux during kVp voltage transitions, i.e., transitions of the output voltage between the first and the second value.

As an example, the triggering scheme may use a comparator that detects when the voltage during a voltage transition is above/below a threshold and trigger detector integration in response. As will be seen below, the present disclosure may also optionally provide a timer that is initiated during voltage transition, e.g., during transition to a high kVp stage. The end of the timer may trigger the next voltage transition, e.g., the falling transition from the high kVp to the low kVp stage.

The voltage generator of the present disclosure may be a voltage generator for providing operating power for an X-ray imaging system, in particular for the radiation source of the X-ray imaging system. The voltage generator may be a high voltage generator. It is noted that a voltage generator is not limited to the element that generates the output voltage, but may also comprise one or more components, e.g., software and/or hardware components, implementing other functions, including, but not limited to, the functions of the trigger system.

The voltage generator may be configured to output power according to an output power (kVp) switching scheme. The voltage generator may be configured to transition, in particular alternate, between two output voltage values, in particular, the first value and a second value.

According to the present disclosure, the first value may be a high voltage value and the second value may be a low voltage value, or vice versa. Accordingly, in particular, transitioning to the first value may comprise transitioning, for example, rising or falling, from said second value to the first value.

In particular, the voltage generator may be configured to transition to the first value, keep the output voltage at the first value, subsequently transition to the second value and keep the output voltage at the second value. This may be repeated.

According to the present disclosure, the transitioning is triggered in response to receiving a start cycle trigger.

According to the present disclosure, the start cycle trigger may be an angular trigger, e.g., obtained from an angular encoder, e.g., of a CT-gantry position during rotation. This may, for example, ensure that the angular position of each projection is well-defined and may simplify image reconstruction. Due to variations of rotation speed, cycles may have different lengths. Alternatively to using a start cycle trigger from an angular encoder, a periodical trigger may be used.

As can be seen from the above, the transitioning is triggered in response to receiving the start cycle trigger and the data acquisition cycle, particularly the first integration period thereof, may thus be triggered in response to the output voltage actually transitioning, more specifically, in response to the output voltage passing a predetermined threshold. Accordingly, the start cycle trigger may thus cause or dictate the start of the data acquisition cycle of the detector. At the same time, the integration periods may be coupled closely to the voltage transitions.

It is noted that due to coupling the integration period to the output voltage, a certain degree of decoupling from the start cycle trigger is caused. Particularly, this means that a correction that accounts for the data acquisition not being exactly synchronized with the start cycle trigger may be provided. For example, when the start cycle trigger is an angular encoder trigger, the orientation associated with the integration period may have an offset and a correction for this offset may be provided.

A correction may be accomplished, for example, by detecting a time difference between the start cycle trigger (angular encoder trigger) and the trigger triggering the start of the data acquisition for the integration period. This time difference can be used to estimate the angular mismatch between the nominal angle of the encoder trigger and the real gantry angle for the start of the data acquisition. This estimated angular mismatch can be used in the reconstruction to correct for the angular jitter. Alternatively, correction may be performed by means of an adaptive digital phase-locked loop, A-DPLL, which models a rotation, e.g., of a gantry to which the angular encoder is attached. An A-DPLL trigger that may be used is explained in detail in the European patent application EP21216352.1, which is incorporated herein by reference. The A-DPLL can be used to improve the angular accuracy of the trigger signals from the angular encoder. Within the A-DPLL the gantry angle is estimated, and the actual value taken at the acquisition trigger time can be used in the image reconstruction to correct for the angular jitter.

As briefly mentioned above, the trigger system according to the present disclosure may be configured to start a timer with a predetermined time interval in response to the determining that the output voltage passes the predetermined threshold. That is, the timer may be started at the time when the first integration period is started.

According to the present disclosure, the trigger system may further be configured to, in response to expiry of the timer, output a transition trigger configured to trigger the voltage generator to start transitioning from the first value to a second value of the output voltage. In particular, the first value may be higher than the second value.

Using a timer as disclosed herein allows for further reducing jitter. In particular, when the first value is the high voltage value, jitter can be effectively reduced for the high voltage period by means of the timer. Any remaining integral jitter including, for example, the cycle length jitter, will then occur in and essentially be consumed by the low voltage period, which is less sensitive than the high voltage period.

According to the present disclosure, the threshold may be a first threshold and the trigger system may further be configured to, subsequently to triggering the start of data acquisition for the first integration period and in response to determining that the output voltage passes a predetermined second threshold, trigger the end of the data acquisition of the first integration period. Optionally, the trigger system may further be configured to, subsequently to triggering the start of data acquisition for the first integration period and in response to determining that the output voltage passes the predetermined second threshold, trigger a start of a data acquisition for a second integration period of the data acquisition cycle.

In particular, the second integration period may start at the same time the first integration period ends.

By using the second threshold, the second integration period may reliably be synchronized with the voltage transition, thereby reducing adverse effects due to jitter.

The first threshold may be the same as the second threshold. Alternatively, the first threshold may be different from the second threshold. Each threshold may be selected based on steepness of the respective transition and/or so as to minimize the detrimental effect of jitter.

For example, since the transitions may be different in steepness and/or since jitter may be more or less detrimental in the high voltage or low voltage period, it may be advantageous to provide different thresholds.

According to the present disclosure, transitioning to the first value of the output voltage may comprise increasing the output voltage. Triggering the start of the data acquisition of the detector for the first integration period of the data acquisition cycle may be performed in response to determining that the output voltage exceeds the threshold, in particular, the first threshold. Thus, the data acquisition cycle may start with a high voltage period and the first integration period may be associated with the high voltage period.

Alternatively or in addition, according to the present disclosure, transitioning from the first value to a/the second value of the output voltage may comprise decreasing the output voltage. Triggering a/the end of the data acquisition of the first integration period, and optionally, triggering a/the start of a/the data acquisition for a/the second integration period of the data acquisition cycle, may be performed in response to determining that the output voltage falls below a/the predetermined second threshold. Thus, the second integration period may be associated with a low voltage period.

A data acquisition cycle may comprise at least a first and a second integration period, and associated therewith, a high voltage period and a low voltage period. The second integration period may immediately follow the first integration period. Accordingly, the high and low voltage periods may immediately follow each other. A data acquisition cycle may optionally comprise more than two integration periods.

Similar to the determination of the gantry angle for the start of the first data acquisition, the trigger for the second data acquisition can be used to determine the gantry angle for the reconstruction. As described above, either a time measurement or the modeled angle of an A-DPLL can be used to estimate the gantry angle for the second data acquisition. This correction will reduce the angular jitter for the reconstruction.

The voltage generator may be comprised in an imaging system, e.g., an X-ray imaging system, in particular, may be comprised in or connected to an X-ray source so as to provide operating power to the X-ray source.

The invention also provides an X-ray imaging system comprising a trigger system configured to, in response to receiving a start cycle trigger, trigger a voltage generator to start transitioning to a first value of an output voltage of the voltage generator. The trigger system is further configured to, in response to determining that the output voltage passes a predetermined threshold, trigger a start of a data acquisition of a detector for a first integration period of a data acquisition cycle of the detector.

The voltage generator may be part of or connected to the X-ray imaging system. The voltage generator may be a voltage generator configured for powering an X-ray source of the X-ray imaging system, in particular a voltage generator connected to the X-ray source or comprised in the X-ray source.

The advantages and features outlined above in the context of the voltage generator similarly apply to the X-ray imaging system. In particular, the trigger system may be any of the above-described trigger systems.

The trigger system may be part of the voltage generator (as described above) or it may be separate from and in signal communication with the voltage generator.

The trigger system according to the present disclosure may further be configured to start a timer with a predetermined time interval in response to the determining that the output voltage passes the predetermined threshold. In particular, according to the present disclosure, the trigger system may further be configured to, in response to expiry of the timer, output a transition trigger configured to trigger the voltage generator to start transitioning from the first value to a second value of the output voltage.

According to the present disclosure, the threshold may be a first threshold and the trigger system may further be configured to, subsequently to triggering the start of data acquisition for the first integration period and in response to determining that the output voltage passes a predetermined second threshold, trigger the end of the data acquisition of the first integration period. Optionally, the trigger system may further be configured to, subsequently to triggering the start of data acquisition for the first integration period and in response to determining that the output voltage passes the predetermined second threshold, trigger a start of a data acquisition for a second integration period of the data acquisition cycle. The first threshold may be the same as the second threshold. Alternatively, the first threshold may be different from the second threshold.

According to the present disclosure, transitioning to the first value of the output voltage may comprise increasing the output voltage and triggering the start of the data acquisition of the detector for the first integration period of the data acquisition cycle may be performed in response to determining that the output voltage exceeds the threshold, in particular, the first threshold.

Alternatively or in addition, according to the present disclosure, transitioning from the first value to a/the second value of the output voltage may comprise decreasing the output voltage and triggering a/the end of the data acquisition of the first integration period, and optionally, triggering a/the start of a/the data acquisition for a/the second integration period of the data acquisition cycle, may be performed in response to determining that the output voltage falls below a/the predetermined second threshold.

The X-ray imaging system according to the present disclosure may comprise a voltage generator, particularly a voltage generator as described above or specified in the claims. As briefly mentioned above, the trigger system may in part or fully be included in the voltage generator or may be external to the voltage generator.

Alternatively or in addition, the X-ray imaging system according to the present disclosure may comprise an X-ray source, particularly comprising the voltage generator as described above or specified in the claims. The X-ray source may be configured such that the output voltage of the voltage generator provides the operating power for the X-ray source.

Alternatively or in addition, the X-ray imaging system according to the present disclosure may comprise a detector, wherein the detector is configured to perform the data acquisition. The detector may be any detector suitable for X-ray imaging. The detector may be configured to perform data acquisition in accordance with the triggers provided by the trigger system.

Alternatively or in addition, the X-ray imaging system according to the present disclosure may comprise a voltage detection system configured to detect the output voltage. The voltage detection system may be configured to output a value of the output voltage, e.g., to a comparator.

Alternatively or in addition, the X-ray imaging system according to the present disclosure may comprise a comparator configured to detect that the output voltage passes the predetermined threshold, in particular the first threshold and/or the second threshold, and, in response thereto, trigger the detector to start data acquisition for an integration period, in particular for the first integration period and/or for the second integration period, and optionally to trigger a/the timer. The comparator may receive a value of the output voltage from the voltage detection system, for example.

The invention also provides a method for operating a voltage generator for an X-ray imaging system and/or an X-ray imaging system, in particular the voltage generator and/or X-ray imaging system as described above or specified in the claims. The method comprises, in response to receiving a start cycle trigger, triggering the voltage generator to start transitioning to a first value of an output voltage of the voltage generator. The method further comprises, in response to determining that the output voltage passes a predetermined threshold, triggering a start of a data acquisition of a detector for a first integration period of a data acquisition cycle of the detector.

The determining may be performed making use of a voltage detection system detecting the output voltage and/or a comparator detecting that the output voltage passes the predetermined threshold.

The method of the present disclosure may comprise, in response to the determining that the output voltage passes the predetermined threshold, starting a timer with a predetermined time interval.

In particular, the method of the present disclosure may comprise, in response to expiry of the timer, outputting a transition trigger configured to trigger the voltage generator to start transitioning from the first value to a second value of the output voltage.

The threshold may be a first threshold and the method may further comprise, subsequently to triggering the start of data acquisition for the first integration period, in response to determining that the output voltage passes a predetermined second threshold, triggering the end of the data acquisition of the first integration period and optionally, triggering a start of a data acquisition for a second integration period of the data acquisition cycle. The first threshold may be the same as or different from the second threshold.

Transitioning to the first value of the output voltage may comprise increasing the output voltage and wherein the triggering the start of the data acquisition of the detector for the first integration period of the data acquisition cycle may be performed in response to determining that the output voltage exceeds the threshold, in particular, the first threshold.

Alternatively or in addition, transitioning from the first value to a/the second value of the output voltage may comprise decreasing the output voltage, in particular, wherein triggering a/the end of the data acquisition of the first integration period, and optionally, triggering a/the start of a/the data acquisition for a/the second integration period of the data acquisition cycle, may be performed in response to determining that the output voltage falls below a/the predetermined second threshold.

The invention also provides a computer program product comprising instructions which, when the program is executed by a computer, cause the computer to carry out one or more of the method steps described in the present disclosure.

The invention also provides a computer readable medium comprising instructions which, when executed by a computer, cause the computer to carry out one or more of the method steps described in the present disclosure.

The features and advantages outlined in the context of the voltage generator similarly apply to the X-ray imaging system, the method, the computer program product, and the computer readable medium of the present disclosure.

Further features, examples, and advantages will become apparent from the detailed description making reference to the accompanying drawings.

### BRIEF DESCRIPTION OF THE DRAWINGS

Fig. 1 shows a schematic and not to scale illustration of a voltage generator according to the present disclosure;
Fig. 2 shows a schematic and not to scale illustration of an X-ray imaging system according to the present disclosure;
Fig. 3 shows a flow diagram of a method according to the present disclosure; and
Fig. 4 shows a timing diagram according to the present disclosure.

### DETAILED DESCRIPTION OF EMBODIMENTS

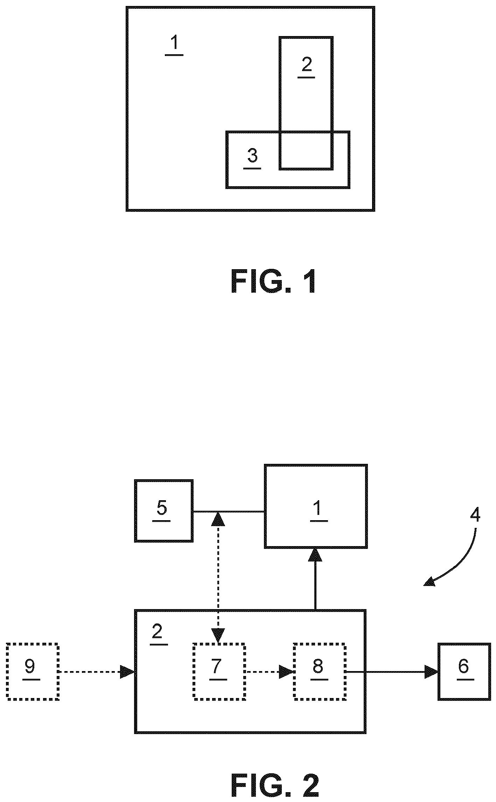

Fig. 1 shows a schematic and not to scale illustration of a voltage generator 1, specifically a high voltage, HV, generator according to the present disclosure. It comprises a trigger system 2, the trigger system configured to, in response to receiving a start cycle trigger, trigger the voltage generator to start transitioning to a first value of an output voltage of the voltage generator. The trigger system is further configured to trigger, in response to determining that the output voltage passes a predetermined threshold, a start of a data acquisition of the detector for a first integration period of the data acquisition cycle. The start cycle trigger, thus, may cause the start of a data acquisition cycle of a detector.

The trigger system may at least in part be implemented making use of a data processing system 3, e.g., comprising one or more computing devices. In particular, any determination steps may be carried out by means of the data processing system and/or one or more of the triggering steps may be carried out by means of the data processing system.

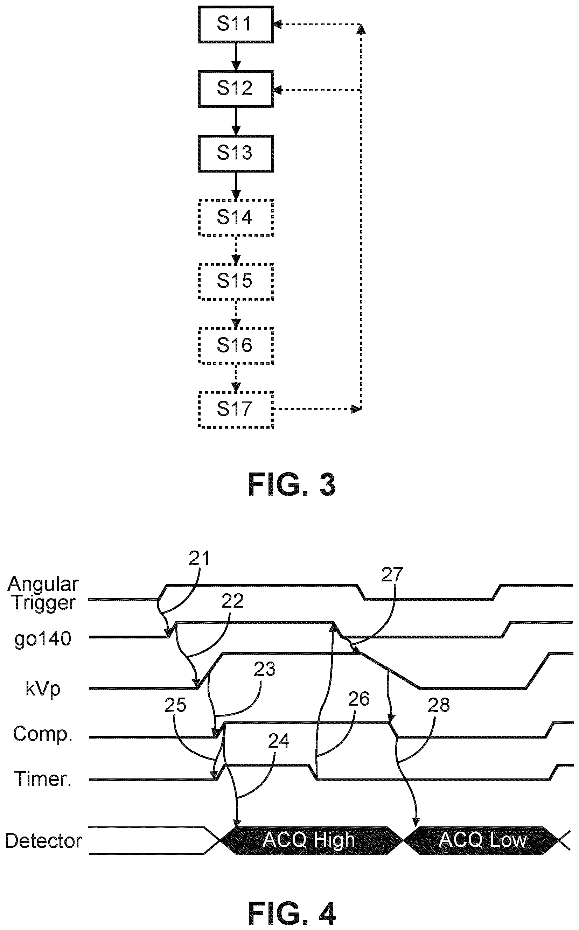

The voltage generator, specifically the trigger system, may be configured carry out any of the methods according to the present disclosure, particularly as shown in Fig. 3. It may be configured to operate according to the timing diagram as shown Fig. 4, for example. The trigger system, in particular the entire voltage generator, may be comprised in or connected to any of the X-ray imaging systems according to the present disclosure, particularly as shown in Fig. 2. Particularly, the voltage generator may be comprised in or connected to an X-ray source of an X-ray imaging system according to the present disclosure.

Fig. 2 shows a schematic and not to scale illustration of an X-ray imaging system 4 according to the present disclosure. The X-ray imaging system comprises a voltage generator 1. The X-ray imaging system comprises a trigger system 2 according to the present disclosure, for example a trigger system as described in the context of Fig. 1. The trigger system may be external to the voltage generator as illustrated in this example or it may be part of the voltage generator, e.g., as shown in Fig. 1.

The X-ray imaging system illustrated in Fig. 2 also comprises an optional X-ray source 5, the X-ray source and voltage generator being configured such that the voltage generator provides operating power to the X-ray source. In Fig. 2, the voltage generator is shown as separate from the X-ray source, but it may alternatively also be incorporated in the X-ray source.

In Fig. 2, the X-ray imaging system also comprises an optional detector 6 that is configured to perform the data acquisition for X-ray imaging, e.g., as instructed by the trigger system.

The X-ray imaging system shown in Fig. 2 also comprises an optional voltage detection system 7 configured to detect the output voltage. For example, this may allow for the trigger system to determine whether or not the output voltage passes the predetermined threshold. To that end, the voltage detected by the voltage detection system may be compared to the threshold.

The X-ray imaging system shown in Fig. 2 also comprises an optional comparator 8 configured to detect that the output voltage passes the predetermined threshold, and, in response thereto, trigger the detector to start data acquisition for an integration period. The comparator may be part of the trigger system.

The comparator may be implemented fully in hardware or fully in software or in a combination of hardware and software. The comparator may be configured such that it detects that the output voltage passes a first threshold and such that it detects that the output voltage passes a second threshold different from the first threshold. Alternatively, two comparators, one for each of the first and second thresholds, may be provided.

The comparator may be configured such that it determines that the output voltage exceeds or falls below the threshold or thresholds.

Optionally, the comparator may be configured to also trigger a timer in response to determining that the output voltage passes the threshold or one or more of the thresholds.

The X-ray imaging system may also comprise an optional angular encoder 9, which may output an angular trigger serving as a start cycle trigger.

Fig. 3 shows a flow diagram of a method for operating a voltage generator for an X-ray imaging system according to the present disclosure and/or for operating an X-ray imaging system according to the present disclosure. For example, the voltage generator or X-ray imaging system may be a voltage generator or X-ray imaging system as shown in and described above in the context of Fig. 1 or Fig. 2 or any other suitable voltage generator or X-ray imaging system, e.g., according to the present disclosure. The method may, for example, implement a timing diagram as shown in Fig. 4.

In step S11, a start cycle trigger is received, e.g., by the trigger system. For example, the start cycle trigger may be an angular trigger received from an angular encoder. The start cycle trigger triggers the voltage generator to start transitioning to a first value of an output voltage of the voltage generator. For example, the voltage generator may be triggered to start increasing the output voltage to a high value kVp_{H}.

In step S12, it is determined that the output voltage passes a (first) predetermined threshold, for example by means of a comparator. To that end, the output voltage may be detected by a voltage detection system, and the detected voltage may be input into the comparator. For example, it may be determined that the output voltage exceeds the (first) predetermined threshold.

In step S13, data acquisition of a detector for a first integration period of the data acquisition cycle is started in response to determining that the output voltage passes the (first) predetermined threshold. To that end, for example, the comparator may output a trigger signal when the detected voltage passes the predetermined threshold.

In optional step S14, a timer is started when the output voltage passes the predetermined (first) threshold.

In optional step S15, for example in response to the expiry of the timer or some other trigger event, a transition trigger triggers the voltage generator to start transitioning from the first value to a second value of the output voltage. For example, the voltage generator may be triggered to start decreasing the output voltage to a low value kVp_{L}.

In optional step S 16, it is determined that the output voltage passes a (second) predetermined threshold, which may be the same or different from the (first) threshold of step S12, for example by means of the comparator. To that end, the output voltage may be detected by the voltage detection system, and the detected voltage may be input into the comparator. For example, it may be determined that the output voltage falls below the (second) predetermined threshold.

In optional step S17, data acquisition of a detector for a second integration period of the data acquisition cycle is started in response to determining, in step S 16, that the output voltage passes the (second) predetermined threshold. To that end, for example, the comparator may output a trigger signal when the detected voltage passes the (second) predetermined threshold.

Optionally, step S12 and S13, and optionally one or more of steps S 14 to S17, may be repeated one or more times.

The method may return to step S11, which marks the beginning of another data acquisition cycle, and some or all of the above-described steps may be repeated for said data acquisition cycle. For example, the method my return to step S11 after step S17, i.e., after at least a second integration period has been started, as indicated in Fig. 3.

Fig. 4 shows an exemplary timing diagram representative of the method of the present disclosure.

A start cycle trigger, for example an angular trigger provided based on an angular encoder signal, triggers the voltage source to start increasing the output voltage (arrow 21). This causes (arrow 22) the output voltage kVp to increase, in particular to transition to (and keep) the high output voltage. The value of the output voltage is input into a comparator (arrow 23). The comparator detects that the output voltage exceeds a threshold and, in response, triggers the start of data acquisition by the detector (arrow 24) for a first integration period and optionally the start of a timer (arrow 25). In response to the timer expiring (or some other trigger event), the voltage generator is triggered to decrease the output voltage (arrow 26), which causes the output voltage to decrease (arrow 27). The comparator detects that the output voltage falls below a threshold and, in response, triggers the start of data acquisition by the detector (arrow 28) for a second integration period. The first integration period is labelled "ACQ High" in this example, as it is performed while the output voltage is high, the second acquisition period is labeled "ACQ Low", as the output voltage is low. In a next step, for example triggered by a second start cycle trigger, the start of another first acquisition period may be triggered, for example in the manner described above. This may also end the preceding second acquisition period. Accordingly, a first and a second acquisition period may together form one acquisition cycle.

However, deviations from such a trigger scheme may be possible. For example, a cycle may comprise more than just two acquisition periods. Moreover, a cycle may start with a decrease of the output signal and, accordingly, with a low voltage acquisition period "ACQ Low". Moreover, alternative trigger events, i.e., other than a timer triggered by the comparator, may be used for ending the first and starting the second acquisition period.

As already explained in other contexts above, the threshold that needs to be passed, e.g. exceeded, by the output voltage to trigger the start of the data acquisition for the first integration period may be different from the threshold that needs to be passed by the output voltage to stop the data acquisition of the first integration period and/or trigger the start of the data acquisition for the second integration period.

While the invention has been illustrated and described in detail in the drawings and foregoing description, such illustration and description are to be considered exemplary and not restrictive. The invention is not limited to the disclosed embodiments. In view of the foregoing description and drawings it will be evident to a person skilled in the art that various modifications may be made within the scope of the invention, as defined by the claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention may be implemented by means of hardware comprising several distinct elements, and/or by means of a suitably programmed processor. In the device claim enumerating several means, several of these means may be embodied by one and the same item of hardware. Measures recited in mutually different dependent claims may advantageously be used in combination.

### LIST OF REFERENCE SIGNS:

| | |
|---|---|
| Voltage generator | 1 |
| Trigger system | 2 |
| Data processing system | 3 |
| X-ray imaging system | 4 |
| X-ray source | 5 |
| Detector | 6 |
| Voltage detection system | 7 |
| Comparator | 8 |
| Angular encoder | 9 |
| Steps | S11 to S17 |
| Arrows | 21 to 28 |

## Claims

1. A voltage generator (1) for an X-ray imaging system (4), the voltage generator (1) comprising a trigger system (2) configured to:
in response to receiving a start cycle trigger, trigger the voltage generator (1) to start transitioning to a first value of an output voltage of the voltage generator (1); and
in response to determining that the output voltage passes a predetermined threshold, trigger a start of a data acquisition of a detector (6) for a first integration period of a data acquisition cycle of the detector (6).

2. The voltage generator (1) of claim 1, the trigger system (2) further configured to, in response to the determining that the output voltage passes the predetermined threshold, start a timer with a predetermined time interval.

3. The voltage generator (1) of claim 2, the trigger system (2) further configured to, in response to expiry of the timer, output a transition trigger configured to trigger the voltage generator (1) to start transitioning from the first value to a second value of the output voltage.

4. The voltage generator (1) of any of the preceding claims, wherein the threshold is a first threshold and the trigger system (2) is further configured to, subsequently to triggering the start of data acquisition for the first integration period, in response to determining that the output voltage passes a predetermined second threshold, trigger an end of the data acquisition of the first integration period and optionally, trigger a start of a data acquisition for a second integration period of the data acquisition cycle, in particular, wherein the first threshold is different from the second threshold.

5. The voltage generator (1) of any of the preceding claims,
wherein transitioning to the first value of the output voltage comprises increasing the output voltage and wherein the triggering the start of the data acquisition of the detector (6) for the first integration period of the data acquisition cycle is performed in response to determining that the output voltage exceeds the threshold, in particular, the first threshold, and/or
wherein transitioning from the first value to a/the second value of the output voltage comprises decreasing the output voltage, in particular, wherein triggering an/the end of the data acquisition of the first integration period, and optionally, triggering a/the start of a/the data acquisition for a/the second integration period of the data acquisition cycle, is performed in response to determining that the output voltage falls below a/the predetermined second threshold.

6. An X-ray imaging system (4) comprising:
the voltage generator (1) according to any one of claims 1 to 5,
an X-ray source (5),
wherein the trigger system comprises:
a voltage detection system (7) configured to detect the output voltage, and/or
a comparator (8) configured to detect that the output voltage passes the predetermined threshold, in particular the first threshold and/or the second threshold, and, in response thereto, trigger the detector (6) to start data acquisition for an integration period, in particular for the first integration period and/or for the second integration period, and optionally to trigger a/the timer.

7. A method for operating a voltage generator (1) for an X-ray imaging system (4), in particular the voltage generator (1) of any one of claims 1 to 5, and/or for operating an X-ray imaging system (4), in particular the X-ray imaging system (4) of claim 6, the method comprising:
in response to receiving a start cycle trigger, trigger the voltage generator (1) to start transitioning to a first value of an output voltage of the voltage generator (1); and
in response to determining that the output voltage passes a predetermined threshold, triggering a start of a data acquisition of a detector (6) for a first integration period of a data acquisition cycle of the detector (6),
in particular, the determining being performed making use of a voltage detection system (7) detecting the output voltage and/or a comparator (8) detecting that the output voltage passes the predetermined threshold.

8. The method of claim 7, further comprising, in response to the determining that the output voltage passes the predetermined threshold, starting a timer with a predetermined time interval.

9. The method of claim 8, further comprising, in response to expiry of the timer, outputting a transition trigger configured to trigger the voltage generator (1) to start transitioning from the first value to a second value of the output voltage.

10. The method of any one of claims 7 to 9, wherein the threshold is a first threshold and the method further comprises, subsequently to triggering the start of data acquisition for the first integration period, in response to determining that the output voltage passes a predetermined second threshold, triggering the end of the data acquisition of the first integration period and optionally, triggering a start of a data acquisition for a second integration period of the data acquisition cycle, in particular, wherein the first threshold is different from the second threshold.

11. The method of any one of claims 7 to 10,
wherein transitioning to the first value of the output voltage comprises increasing the output voltage and wherein the triggering the start of the data acquisition of the detector (6) for the first integration period of the data acquisition cycle is performed in response to determining that the output voltage exceeds the threshold, in particular, the first threshold, and/or
wherein transitioning from the first value to a/the second value of the output voltage comprises decreasing the output voltage, in particular, wherein triggering a/the end of the data acquisition of the first integration period, and optionally, triggering a/the start of a/the data acquisition for a/the second integration period of the data acquisition cycle, is performed in response to determining that the output voltage falls below a/the predetermined second threshold.

12. A computer program product comprising instructions which, when the program is executed by a computer, cause the computer to carry out the method of any one of claims 7 to 11.

13. A computer readable medium comprising the computer program product of claim 12.
